# EUROPEAN PATENT APPLICATION

(11) **EP 4 275 579 A1**
(43) Date of publication of application: **15.11.2023**
(21) Application number: 22173325.6
(22) Date of filing: 13.05.2022
(51) Int. Cl.: A61B 1/00, A61B 1/04, A61B 1/06, G06T 7/00, A61B 5/00

(54) **METHOD, PROCESSOR, AND MEDICAL OBSERVATION DEVICE USING TWO COLOR IMAGES AND COLOR CAMERAS FOR FLUORESCENCE AND WHITE-LIGHT**

(71) Applicant: Leica Instruments (Singapore) Pte. Ltd., Singapore 608924 (SG)
(72) Inventor: Themelis, George, 88131 Lindau (DE); Pentarakis, Kyriakos, 9434 Au (CH)
(74) Representative: Grünecker Patent- und Rechtsanwälte PartG mbB

(57) **Abstract**

This application relates to an image processor (170) and a computer-implemented image processing method for generating a digital output color image (160) of an object (106) using a medical observation device (100), such as a microscope or endoscope. The method comprises the steps of recording or retrieving a digital white-light color image (114) of the object (106) in a first imaged spectrum (202) using a white-light color camera (110); recording or retrieving a digital fluorescence-light color image (112) of the object (106) in a second imaged spectrum (222) using a fluorescence-light color camera (111), the second imaged spectrum (222) overlapping a fluorescence emission spectrum (226) of at least one fluorophore (116), the second imaged spectrum (222) being different from the first imaged spectrum (202), the first and the second imaged spectrum (202, 222) both overlapping the visible spectrum (212); and generating the digital output color image (160) by combining the digital fluorescence-light color image (112) and the digital white-light color image (114). The image processor (170) is configured to carry out these steps. The application further relates to a medical observation device (100) comprising such an image processor (170) and a method of operating such a device including the above computer-implemented method.

## Description

The claimed subject matter relates to a computer-implemented image processing method and to an image processor for generating a digital output color image of an object using a medical observation device, such as a microscope or endoscope. The claimed subject matter further relates to a method for operating such a medical observation device and to a medical observation device itself.

In existing medical observation devices, fluorescence and white-light images are recorded by a single camera. The problem, however, is that fluorescence intensity is much lower than the white-light reflectance. The recorded fluorescence is thus hard to detect as it is drowned by the white-light reflectance.

Other medical observation devices use a gray scale camera to record fluorescence and a color camera to record the white-light image. In such a set-up, the fluorescence image and the white-light image are digitally mixed, and fluorescence is marked with pseudocolor. Here, the disadvantage is that the pseudocolor does not accurately represent the colors of fluorescence as they would be perceived by the human eye.

There is therefore a need to provide a medical observation apparatus which generates a natural-looking, i.e. true-color, image of both the reflected spectrum and the fluorescence spectrum of an object.

This need is addressed by a computer-implemented image processing method for generating a digital output color image of an object, in particular using a medical observation device such as a microscope or endoscope, the method comprising the steps of: retrieving a digital white-light color image of the object recorded in a first imaged spectrum e.g. using a white-light color camera; retrieving a digital fluorescence-light color image of the object recorded in a second imaged spectrum e.g. using a fluorescence-light color camera, the second imaged spectrum overlapping a fluorescence emission spectrum of at least one fluorophore, the second imaged spectrum being different from the first imaged spectrum, the first and the second imaged spectrum both overlapping the visible spectrum; and generating the digital output color image by combining the digital fluorescence-light color image and the digital white-light color image.

This need is further addressed by an image processor for a medical observation device, such as a microscope or endoscope, the image processor being configured to retrieve a digital white-light color image of an object recorded in a first imaged spectrum e.g. using a white-light color camera; retrieve a digital fluorescence-light color image of the object recorded in a second imaged spectrum e.g. using a fluorescence-light color camera, the first imaged spectrum overlapping a fluorescence emission spectrum of at least one fluorophore, the first imaged spectrum being different from the second imaged spectrum, the first and the second imaged spectrum both overlapping the visible spectrum; and generating a digital output color image by combining the digital fluorescence-light color image and the digital white-light color image.

Finally, the need is addressed by use of a fluorescence-light color camera in a medical observation device to record part of the spectrum of a digital output color image generated by the medical observation device, the medical observation device further comprising a white-light color camera configured to record another part of the spectrum of the digital output color image.

By employing a color camera for both the fluorescence and the white-light image, a much more color-accurate, true-color output image may be provided.

The above solutions may be further improved by the following features, which may be added and combined independently of one another, and each of which has its own beneficial technical effect.

Each of the following features may be used both for improving one of the above methods and/or for improving one of the above devices, independent of whether the particular feature is only mentioned in relation to a method or in relation to a device.

The computer-implemented image processing method may include the step of demosaicing at least one of the digital white-light color image and the digital fluorescence-light color image. By demosaicing, color artifacts may be avoided.

The computer-implemented image processing method may further include the step of normalizing at least one of the digital white-light color image and the digital fluorescence-light color image. Normalization facilitates the joint processing of the digital white-light color image and the digital fluorescence-light color image.

The computer-implemented image processing method may further include the step of registering at least one of the digital white-light color image and the digital fluorescence-light color image. By registering, image features that are present in both the digital white-light color image and the digital fluorescence-light color image are represented in each image by the same size and orientation. After registering, the digital white-light color image and the digital fluorescence-light color image match each other.

The image processor may be configured to carry out any of the above processing steps.

The fluorescence-light color camera is not restricted to recording only fluorescence images. The fluorescence-light color camera may, in one embodiment, record a further digital white-light color image which may complement the digital white-light color image recorded by the white-light color camera. If the second imaged spectrum needs to be changed, i.e. the fluorescence-light color camera is needed to record images in a different second imaged spectrum, a change in the optical set-up may be necessary, e.g. by one or more filters need to be exchanged. This may be the case if a different fluorophore is used in the object.

According to another advantageous embodiment of the computer-implemented image processing method and/or the image processor, the first imaged spectrum and the second imaged spectrum are complementary to each other. In such a configuration, crosstalk between fluorescence-light and white-light is avoided and an exact rendition of the fluorescence of the at least one fluorophore on one hand, and the reflectance of the object on the other, is maintained. Further, by having complementary spectra in the digital fluorescence-light color image and the digital white-light color image, respectively, the joint processing of these two images to form a multispectral image is facilitated.

The digital white-light color image may be represented in a first color space using at least three first color bands.

The digital fluorescence-light color image may be represented in a second color space comprising at least three second color bands. The first and the second color spaces are preferably the same, such that the first and second color bands are also the same. However, this does not need to be the case. The first and the second color spaces can also be different.

The digital output color image may be generated in a third color space comprising at least three third color bands. The third color space may be the same color space as the first and/or the second color space, or be a different color space.

Any of the first, second, and/or third color space may be an RGB color space, comprising three color bands R, G, B, or any other color space, such as multispectral color space or a hyperspectral color space, i.e. employ more than three color bands. The expression "color band" is used in the text as a synonym to "color space coordinate". The same color is represented by different color space coordinates in different color spaces.

According to one embodiment of the computer-implemented image processing method and/or the image processor, the first color bands and the second color bands are mapped onto the third color bands of the digital output color image using a linear transformation. The linear transformation may comprise a color conversion matrix or a linear function. To map the first and second color bands to the third color bands, the color conversion matrix may be applied to the first color bands and the second color bands. Preferably, each color band of the first color bands and each color band of the second color bands is input as a separate color band into the linear transformation. In particular, each color band of the first color bands and each color band of the second color bands may be input simultaneously into the linear transformation.

The color conversion matrix or the linear function may reside in a memory of the image processor or the medical observation device.

The color conversion matrix may have a dimension of a number X in one direction times a number Y in another direction. The number X may be the sum of the quantity of the first color bands, i.e. the number of color bands in the first color bands, and the quantity of the second color bands, i.e. the number of color bands in the second color bands. In other words, one dimension of the color conversion matrix may be the sum of the dimension of the color space of the digital fluorescence-light color image and the dimension of the color space of the digital white-light color image. The number Y may be the quantity of color bands in the third color space. In other words, the other dimension of the color conversion matrix may correspond to the dimension of the color space of the digital output color image. For example, if the first, second and third color spaces are each an RGB color space, the color conversion matrix has a dimension 6x3 or 3x6.

Thus, the third color bands, i.e. the color bands of the digital output color image may result from a linear combination of the color bands of the digital white-light color image and the color bands of the digital fluorescence-light color image.

According to one aspect, the digital fluorescence-light color image and the digital white-light color image are processed jointly as a multispectral image of which the color bands are formed or constituted by the complementary color bands of the digital fluorescence-light color image and the digital white-light color image. This leads to an improved color rendition, especially if the fluorescence-light image represents reflected white light that has been recorded in the second imaged spectrum and thus may complement the white-light information in the white-light color image. Using this approach, the white-light information is contained in the color bands of the digital fluorescence-light color image and thus at a finer color granularity than just the digital white-light color image would offer. If the digital fluorescence-light color image represents fluorescence emission, treating the digital white-light color image and the digital fluorescence-light color image as a multispectral image represents a true representation of the object under both reflected white light and fluorescence.

According to another advantageous embodiment, the first imaged spectrum may comprise a first sub-band in a color band of a color space, the second imaged spectrum may comprise a second sub-band in this color band, wherein the first and the second sub-band are preferably complementary to one another. The color space may be the color space of the digital white-light color image, the color space of the digital fluorescence-light color image or, preferably, the color space of the digital output color image. Having such a subdivided color band facilitates the linear transformation for mapping the color bands of the fluorescence-light and the white-light color images to the color bands of the digital output color image. It is preferred that the sub-bands of one color-band, together, complete the respective color band. This ensures that no useful spectral information is lost.

Of course, there may be a first and second sub-band in more than one color band of the respective color space. The more sub-bands in different color bands are used, the more accurate the spectral information recorded in the respective white-light or fluorescence-light color image is. A sub-band in a color band may be, in itself, divided to a plurality of distinct wavelength bands that are separate from each other.

Preferably, the second imaged spectrum comprises an IR wavelength. This allows fluorescence emission in the near infrared to be captured.

The above computer-implemented image processing method may be carried out when executing a method for operating a medical observation device. The image processor as described above, may be part of a medical observation device such as a microscope or endoscope, in particular a microscope or endoscope configured for surgery such as neurosurgery, orthopedic surgery and/or ophthalmic surgery. The microscope may also be a laboratory microscope used, e.g. for biopsy.

The method for operating the medical observation device may comprise the steps of recording the digital white-light color image using the white-light color camera and recording the digital fluorescence-light color image using the digital fluorescence-light color camera. The medical observation device may comprise a digital fluorescence-light color camera and the white-light color camera.

In order to reduce post processing, the fluorescence-light color camera and the white-light color camera may have overlapping coaxial and/or, preferably, identical field of views.

The medical observation device may be a stereoscopic or monoscopic device. In one embodiment, a digital fluorescence-light color camera and a white-light color camera may be provided in each stereoscopic channel. Alternatively, in a monoscopic medical observation device, only a single white-light color camera and a single digital fluorescence-light color camera may be provided.

In an alternative arrangement of a stereoscopic medical observation device, the fluorescence-light color camera may be provided in one stereoscopic channel, whereas the white-light color camera may be provided in the other stereoscopic channel. In such an arrangement, the fluorescence-light color camera may be configured to record (white-light) reflectance images in the second imaged spectrum. For (white-light) reflectance images, this arrangement provides a stereoscopic view, although strictly speaking, the stereoscopic view is limited to the overlapping parts of the first and second imaged spectrum, although this will be rarely noted by a human observer. In cases where fluorescence emission is recorded, the fluorescence-light color camera provides a monoscopic view on the fluorescence emission of the object, whereas the white-light color camera provides a monoscopic view on the reflectance of the object.

The medical observation device may comprise an optical color separation assembly, which is configured to split the light entering color separation assembly into the first imaged spectrum and the second imaged spectrum. The color separation assembly may comprise optical elements such as a beam splitter, in particular a dichroic beam splitter, and/or optical filters such as a fluorescence-light filter blocking light outside the fluorescence emission and a white-light filter blocking the fluorescence emission.

The medical observation device may further comprise an illumination assembly, which is preferably tunable, e.g. by comprising a plurality of differently-colored LEDs or OLEDs or other light sources which emit light in a plurality of different spectral bands and can be turned on and off individually.

To facilitate post processing and to ensure that the recorded intensity values of the digital white-light image and the digital fluorescence-light color image are comparable to one another, it is preferred that the digital fluorescence-light color image and the digital white-light color image are recorded at the same time and/or use the same exposure time and/or use the same gain and/or the same white balance and/or color compensation. Moreover, the gain of the digital fluorescence-light color camera and the gain of the digital white-light color camera may be kept at a constant ratio, but otherwise allowed to adapt automatically. Of these possibilities, it is most preferred that the white-light color camera and the fluorescence-light color camera are synchronized with respect to at least one of exposure time and gain. The white-light color camera and/or the fluorescence-light color camera is preferably a CCD or CMOS camera. The fluorescence-light camera and the white-light color camera are preferably identical.

According to another aspect, the medical observation device may comprise a memory in which a plurality of different color conversion matrices is stored. Each color conversion matrix represents a different combination of a first and a second imaged spectrum, i.e. a different set of filters used in the color separation assembly, as is necessary if different fluorophores or combinations of different fluorophores are used. Examples of fluorophores used in this context are 5-ALA, fluorescein and ICG. Each of these fluorophores requires excitation at different wavelengths and also emits fluorescence at different wavelengths. Thus, in a surgical environment in which ICG is used, the first imaged spectrum and the second imaged spectrum will be different to that of a surgical environment in which 5-ALA is used, requiring a different color conversion matrix. Thus, the sub-bands of the first and second imaged spectrum in the color bands of the respective color spaces are different for each case of a fluorophore.

A color conversion matrix may be automatically or manually selectable from the plurality of color conversion matrices, depending on the fluorophore used. For example, the medical observation device may be configured to automatically detect the configuration or set-up of the color separation assembly and select a color conversion matrix depending on this set-up. If, for example, the optical filters of the color separation assembly are replaced for using 5-ALA, the medical observation device may automatically select a color conversion matrix for this 5-ALA. Of course, this selection can also be done manually or, an automatic selection may be overrode manually.

The claimed subject matter also relates to a computer-readable medium and a computer program comprising instructions to cause a computer to carry out the computer-implemented image processing in any of the above embodiments.

As used herein, the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

In the following, the invention is described exemplarily with reference to embodiments and with reference to the drawings. The combination of features that are shown in the embodiments is not to be considered as limiting. For example, features of the embodiments which have a technical effect as e.g. explained above, that is not needed in a specific application, may be omitted. Conversely, features described above that are not part of an embodiment described below may be added if the technical effect associated with this particular feature is needed in a specific application.

Throughout the description and the drawings, the same reference numerals are used for elements that correspond to each other with respect to function and/or structure.

In the drawings,
- Fig. 1: shows a schematic representation of a medical observation device;
- Fig. 2: shows a schematic representation of a combination of a digital white-light color image and a digital fluorescence-spectrum color image to arrive at a digital output color image;
- Fig. 3: shows a schematic representation of jointly processing a digital white-light color image and a fluorescence-light color image to arrive at a digital output color image;
- Fig. 4: shows a schematic representation of a method for obtaining a digital output color image from a combination of a digital white-light color image and a digital fluorescence-spectrum color image; and
- Fig. 5: shows a schematic illustration of a system configured to perform a method for obtaining a digital output color image from a combination of a digital white-light color image and a digital fluorescence-spectrum color image.

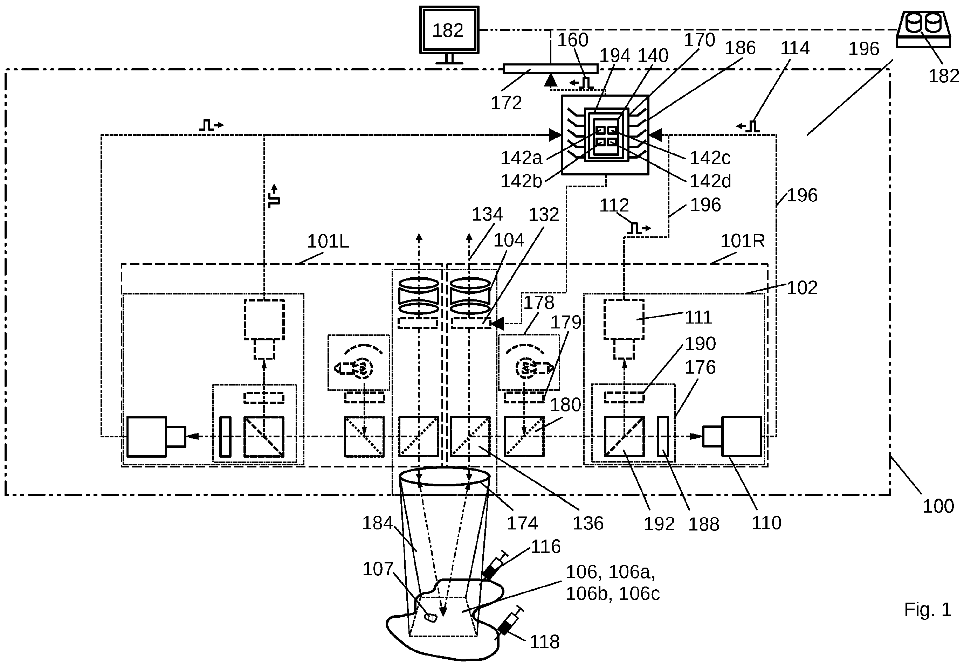

First, an exemplary embodiment of the invention is described with reference to Fig. 1.

Fig. 1 shows schematically a medical observation device 100. The medical observation device 100 may be a microscope or an endoscope, the difference between a microscope and an endoscope being primarily that, in an endoscope (not shown), an object 106 is viewed through optical fibers that are brought into vicinity of the object 106 to be investigated, e.g. by insertion into a body containing the object, whereas, in a microscope, an objective 174 is directed onto the object. Although the medical observation device of Fig. 1 is a microscope, the following description also applies to an endoscope. The medical observation device 100 may be a medical observation device used in surgery. The medical observation device 100 may also be a medical observation device used in a laboratory, such as a laboratory microscope. The object 106 to be investigated may consist of or comprise biological tissue 107.

The object 106 may comprise one or more fluorophores 116, 118. The at least one fluorophore may be a fluorophore that is naturally contained in the object. For example, bone and blood contain fluorophores. The at least one fluorophore may also be added to the object 106, e.g. by injecting it into the biological tissue 107. Examples of fluorophores that may be added to the object 106 are ICG, fluorescein and/or 5-ALA.

The medical observation device 100 is a fluorescence imageing device. That means, that the medical observation device is configured to view and preferably also excite the fluorescence of the one or more fluorophores 116, 118.

The medical observation device 100 may be a stereoscopic device as is exemplarily shown in Fig. 1. It may thus comprise two identical subassemblies 101L and 101R for each of the two stereoscopic channels. As the two subassemblies 101L, 101R are identical with respect to function and structure, the following description focuses on the right subassembly 101R, but applies identically to the left stereoscopic channel 101L.

The medical observation device 100 may alternatively be a monoscopic device. In this case, only one of the two subassemblies 101L, 101R may be present. For a monoscopic medical observation device 100, the following description therefore applies as well.

The medical observation device 100 may be used to generate at least one digital white-light color image 114 which preferably represents a reflectance image of the object 106 across the visible light range. The visible light range or visible spectrum comprises the wavelengths from about 310 nm to about 1100 nm, or from about 380 nm to 750 nm, or from about 450 nm to about 700 nm. The fluorescence spectrum or, if more than one fluorophore is used, the fluorescence spectra of the fluorophores 116, 118 is preferably omitted from the spectrum recorded in the digital white-light color image 114. This makes sure that only reflected light is contained in the digital white-light color image 114 if fluorescence is present. For example, if 5-ALA is used as a fluorophore, the fluorescence spectrum from about 625 nm to about 650 nm may not be recorded in the digital white-light color image 114.

If light of specific wavelengths is used to excite fluorescence, the spectrum comprising or consisting of these wavelengths may also not be recorded or represented in the digital white-light color image 114. For example, if 5-ALA is used as a fluorophore, fluorescence may be excited by illuminating the object 106 with wavelengths between about 380 nm to about 450 nm. For fluorescein and ICG and other fluorophores, different but known ranges for the excitation and emission spectra apply than for 5-ALA.

By not recording the fluorescence-excitation spectrum and the fluorescence-emission spectrum, the digital white-light color image 114 represents the reflectance of the object 106, i.e. represents a white-light image of the object 106 as it would be seen by a human observer. Thus, the digital white-light color image 114 may be regarded as a natural color or true-color image.

The digital imaging system 102 may further be used to generate a digital fluorescence-light color image 112 of the object 106. The digital fluorescence-light color image 112 represents the fluorescence emission of the one or more fluorophores 116, 118. The digital fluorescence-light color image 112 therefore does preferably not record any wavelengths outside the emission spectrum or the emission spectra of the one or more fluorophores.

The digital white-light color image 114 and the digital fluorescence-light color image 112 are both color images. They are recorded using at least three color bands or, equivalently, primary colors of a color space. For example, the digital white-light color image 114 and the digital fluorescence color image 112 may be recorded in RGB color space using the three primary colors or color bands R, G, B. Alternatively, the digital white-light color image 114 and the digital fluorescence color image 112 may be recorded in different color spaces, respectively, and/or represent multispectral or hyperspectral color images. The digital white-light color image 114 and the digital fluorescence color image 112 need not be recorded in the same color space, although this is preferred.

In a color space, such as RGB color space, each color is represented by triple of three integer numbers, wherein each integer number indicates the intensity of one of the primary colors R, G, B. Each number represents a color space coordinate and each color band a coordinate axis. For example, the most intense red is indicated by the triple [255 ,0, 0]. The most intense green color is indicated by [0, 255, 0], and the most intense blue by [0, 0, 255]. Thus, RGB color space is a three-dimensional space, CMYK color space would be a four-dimensional space. A color can be considered as a point in color space to which a vector such as [0, 0, 255] points. A multispectral or hyperspectral color space having n color bands would correspondingly result in an n-dimensional color space, in which each color is represented by an n-tuple of color space coordinates.

The spectrum recorded in the digital white-light color image 114 and the spectrum recorded in the digital fluorescence-light color image 112 are preferably complementary to one another, i.e. do not overlap.

More specifically, the digital imaging system 102 may comprise a digital imaging system 102 for generating the digital fluorescence-light color image 112 and the digital white-light color image 114. The digital imaging system 102 may comprise a white-light color camera 110 and a fluorescence-light color camera 111.

The white-light color camera 110 is configured to record the digital white-light color image 114. In particular, the white-light color camera 110 may be configured to generate a stream of digital white-light color images 114 in the form of a video stream. The white-light color camera 110 is preferably configured to record a digital image across the entire visible spectrum in the wavelengths indicated above. The white-light color camera 110 may be a CCD, CMOS or multispectral or hyperspectral camera.

The fluorescence-light color camera 111 is configured to record the digital fluorescence-light image 112. In particular, the fluorescence-light camera 111 may be configured to generate a stream of digital fluorescence-light color images 112 in the form of a video stream. The fluorescence-light color camera 111 may be configured to record the digital fluorescence-light color image 114 only in the fluorescence spectrum or the fluorescence spectra of the at least one fluorophore 116, 118. The fluorescence-light camera 111 may be configured to record the digital fluorescence-light image only in one or more narrow bands of light. These narrow bands should overlap the fluorescence spectrum or spectra of the one or more fluorophores 116, 118 of which fluorescence is to be recorded. Preferably, the fluorescence spectra of the fluorophore 116 and the second fluorophore 118 are at least partly separate, preferably completely, so that the fluorescence-light camera 111 may record light in two separate fluorescence bands that are spaced from one another.

The fluorescence-light color camera 111 may be a CCD, CMOS or multispectral or hyperspectral camera. Preferably, the white-light color camera 110 and the fluorescence-light color camera 111 are of the same type, although this is not necessary.

The respective fields of view 184 of the cameras 110, 111 are preferably aligned or even coinciding and coaxial. Thus, it is preferred that the cameras 110, 111 provide the identical field of view 184 with the identical perspective and focal length. This results in identical representations of the object 106 in the images 112, 114 generated by the different cameras 110, 111. Both cameras 110, 111 may use the same objective 174.

If a match of the perspectives and field of view cannot be generated optically, it may be generated by image processing by applying a matching or registering routine to the digital images 112, 114, as is explained further below.

It is preferred that the two cameras 110, 111 are operated synchronously. Specifically, the exposure times may be synchronized. Thus, the medical observation device 100 may be configured to generate the digital white-light color image 114 and the digital fluorescence-light image 112 at the same time.

Preferably, the gain of the two cameras 110, 111 is synchronized, i.e. adjusted in the two cameras 110, 111 at the same time. Moreover, the ratio of the gain applied in camera 110 to the gain applied in camera 111 may be constant, even if the gain is changed. The gamma correction and color adjustment or white balance may be switched off or kept constant.

Any of the above measures facilitates the comparison, joint processing and/or combining of the two images 112, 114

For separating the spectrum recorded in the digital white-light color image 114 from the spectrum recorded in the digital fluorescence-light color image 112, i.e. for separating the reflectance spectrum from the fluorescence spectrum, an optical color-separation assembly 176 may be provided. The color-separation assembly 176 may comprise optical elements such as a beam splitter 192, which may be dichroic. The color separation assembly 176 may further or alternatively comprise an optical white-light filter 188 and/or an optical fluorescence-light filter 190.

The fluorescence-light filter 190 is preferably configured to transmit light in the fluorescence spectrum or spectra of the one or more fluorophores 116, 118 and to block light outside the fluorescence spectrum or spectra.

The fluorescence-light filter 190 may be configured as a band-pass filter comprising one or more passbands. Each passband should overlap the fluorescence emission spectrum of a respective fluorophore 116, 118 of which the fluorescence is to be recorded. As the fluorescence-light filter 190 is in the light path between the beam splitter 192 and the fluorescence-light camera 111, only the wavelengths in the passbands of the fluorescence-light filter 190 are transmitted to the fluorescence-light camera 111.

The white-light filter 188 is preferably configured to block light in the fluorescence spectrum or spectra of the one or more fluorophores 116, 118. The white-light filter 188 may also be configured to block light in the fluorescence-excitation spectrum.

The white-light filter 188 is preferably configured as a band-stop filter, of which the stop bands correspond to or at least contain the passbands of the fluorescence-light filter 190. The white-light filter 188 is located in the light path between the beam splitter 192 and the white-light camera 110. Thus, white-light camera 110 records only wavelengths that are outside the stop-bands of the white-light filter 188 and therefore also outside of the pass-bands of the fluorescence-light filter 190 of the fluorescence-light filter 190.

Any one of the white-light filter 188 and the fluorescence-light filter 190 may be a tunable filter.

If the beam splitter 192 is a dichroic beam splitter, at least one of the filters 188, 190 may be omitted as the optical spectral filtering in this case is already integrated in the dichroic beam splitter. The above description of the passbands and stopbands then should apply mutatis mutandis to the dichroic beam splitter 192.

The medical observation device 100 may further comprise an illumination assembly 178, which is configured to illuminate the object 106 preferably through the objective 174 through which the imaging system 102 records the at least one digital image 112, 114. The illumination assembly 178 may be configured to selectively generate white-light, i.e. light that is evenly distributed across the entire visible spectrum, and fluorescence-excitation light, which contains light only in wavelengths that stimulate fluorescence of the at least one fluorophore 116, 118. The illumination light generated by the illumination assembly 178 may be fed into the objective 174 using an illumination beam splitter 180.

An illumination filter 179 may be provided depending on the fluorophore and its fluorescencespecific excitation spectrum. For example, if 5-ALA is used as a fluorophore, the illumination filter may have a transmission of 90 % to 98 % up to wavelengths of 425 nm, a transmission between 0.5 % and 0.7 % in wavelengths between 450 nm and 460 nm, a transmission of not more than 0.1 % between 460 nm and 535 nm and of practically zero for wavelengths above 535 nm.

Instead of or in addition to the illumination filter 179, the illumination assembly 178 may comprise a tunable light source, comprising e.g. a multitude of differently colored LEDs or OLEDs.

The medical observation device 100 may further comprise an image processor 170. The image processor 170 may be a hardware module, such as a microprocessor, or a software module. The image processor 170 may also be a combination of both a hardware module and a software module, for example by using software modules that are configured to be run on a specific processor, such as a vector processor, a floating point graphics processor, a parallel processor and/or on multiple processors. The image processor 170 may be part of a general-purpose computer 186, such as a PC.

The image processor 170 is configured to retrieve the digital white-light color image 114 and the digital fluorescence-light image 112. For example, the image processor 170 may be configured to retrieve the digital white-light color image 114 and the digital fluorescence-light image 112 from a memory 194 and/or directly from the cameras 110, 111. The memory 194 may be part of the image processor 170 or reside elsewhere in the medical observation device 100.

The image processor 170 is further configured to compute a digital output color image 160 from the digital white-light color image 114 and the digital fluorescence-light image 112. The digital output color image 160 is a color image which is represented in a color space. The color space of the digital output color image may be different from the color space of any of the digital white-light color image 114 and the digital fluorescence-light image 112. Preferably, however, the color space of the digital output color image 160 is the same color space as that of the digital white-light color image 114 and the digital fluorescence-light image 112.

In particular, the image processor 170 is configured to compute the digital output color image 160 from the digital white-light color image 114 and the digital fluorescence-light image 112 by using a color conversion function 140. The color conversion function 140 may be any type of function such as a one- or n-dimensional interpolation function. The color conversion function 140 may be stored in the memory 194.

Preferably, the color conversion function 140 is a linear transformation function. Specifically, the color conversion function 140 may be a color conversion matrix 142. One dimension of the color conversion matrix 142 may be the sum of the number of color bands in the digital fluorescence-light color image 112 and of the number of color bands in the digital white-light color image 114, i.e. the sum of the dimensions of the color spaces of the images 112, 114. Another dimension of the color conversion matrix 142 may correspond to the number of color bands in the digital output color image 160, i.e. the dimension of the color space of the digital output color image 160.

If all the images 112, 114, 160 are in RGB color space, the dimension of the color conversion matrix is preferably 6x3 or 3x6.

A specific color conversion matrix 142 may be selectable from a set of color conversion matrices 142a-d stored in the medical observation device 100 or the image processor 170, e.g. the memory 194. For example, different color conversion matrices 142a-d may be stored in memory for different fluorophores.

For example, a first color conversion matrix 142a may represent the case of ICG being used as a fluorophore 116 in the object 106, a second color conversion matrix 142b may represent the case of case of 5-ALA being used as a fluorophore 118 in the object 106 and/or a third color conversion matrix 142c may represent the case of 5-ALA and ICG being used simultaneously as fluorophores 116, 118 in the object 106. Similarly, further color conversion matrices 142d may be used for other fluorophores or combination of fluorophores or combination of fluorophores. In its simplest form, the color conversion matrix 142 performs an addition of the intensities in each color band of the color space of the fluorescence-light color image and the white-light color image separately.

Each different color conversion matrix 142 may represent different filter settings of the color separation assembly 176. As the different fluorophores have different emission spectra, the wavelengths that are to be recorded by the white-light color camera 110 and by the fluorescence-light camera 111 differ for each fluorophore. Thus, for each fluorophore, the color bands in the two cameras 110, 111 are used to a different extent, requiring different calibration matrices for adjusting the relative intensities in the color bands of the images 112, 114.

The medical observation device 100 may be adjusted to a different fluorophore by re-configuring the color-separation assembly 176, e.g. by exchanging its optical elements, such as the filters 190 and/or 192, or the dichroic beam splitter 180 and by selecting the corresponding color conversion matrix 142. The selection of the color conversion matrix 142 may be done automatically depending on the current set-up of the color separation assembly 176 or manually by a user.

The digital output color image 160 may be displayed on a display 132 which is integral with the medical observation device 100. For example, the display 132 may be integrated in an ocular or eyepiece 104 of the medical observation device 100.

The medical observation device 100 may comprise a direct optical path 134 from the object 106 through the objective 174 to the eyepiece 104. In such a case, the display may be a translucent display 132 located in the direct optical path 134 or the display may be projected into the direct optical path 134. A beam splitter 136 may be provided to split the light between the optical eyepiece 104 and the digital imaging system 102. In one embodiment, up to 80 % of the light may be directed to the eyepiece 104.

Alternatively, the medical observation device 100 may not have a direct optical path 134 but only display images from the integral display 132. As a further alternative, the medical observation device may not have any display at all.

The medical observation device 100 may comprise an output interface 172 to which one or more (external) displays 182 may be connected. For this, the output interface 172 may comprise standardized connectors and data transmission protocols, such as USB, HDMI, DVI, DisplayPort, Bluetooth and/or others. An external display may be a monitor, 3D goggles, oculars and the like. Any combination of external displays may be connected to output interface 172.

The computer 186 or the image processor 170 is connected to the digital imaging system 102 using one or more data transmission lines 196. A data transmission line may be wired or wireless, or partly wired and partly wireless. The computer 186 and/or the image processor 170 may not be bodily integrated in the medical observation device 100 but be physically located remote from the digital imaging system 102. For this, the digital imaging system 102 and the computer 186 and/or the image processor 170 may be connected to a network, such as a LAN, a WLAN or a WAN, to which also at least one display 182 is connected.

According to a modification, the medical observation device 100 may be stereoscopic but comprise only two cameras, one for each stereoscopic channel. In one stereoscopic channel, the fluorescence-light color camera 111 is used and configured to selectively also record white-light reflectance, whereas in the other stereoscopic channel, the white-light color camera 110 is used.

Such an arrangement provides a stereoscopic white-light color image if no fluorescence is used and a monoscopic white-light color image and a monoscopic fluorescence-light color image if fluorescence is used. The description above and below applies equally to this configuration.

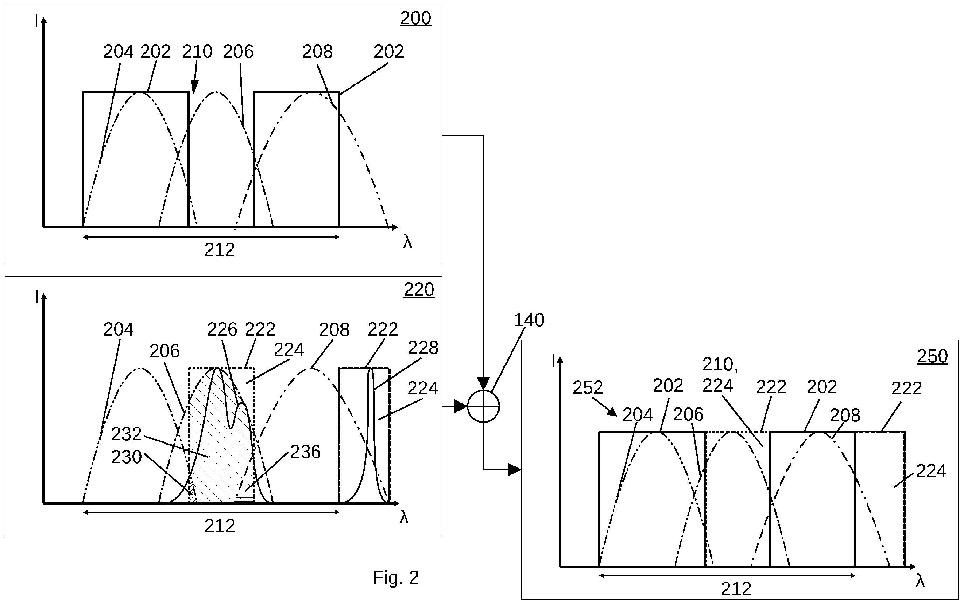

Fig. 2 shows at reference numeral 200 a quantitative example of a first imaged spectrum 202 as it may be recorded by the digital white-light camera 110 and/or represented in the digital white-light color image 114, respectively. The intensity I across the wavelengths/colors A is shown as normalized. The first imaged spectrum 202 preferably extends at least across the visible spectrum 212.

Just by way of example, the color space in which the first imaged spectrum 202 is recorded, is an RGB color space having three primary colors or color bands 204, 206 and 208. One primary color 204 is blue, another primary color 206 is green and a third primary color 208 is red. The sensitivities of the sensors of the white-light color camera 110 in the different primary colors 204, 206, 208 are tuned to result in a sensitivity across the visible spectrum 212 which is as constant as possible.

If a color space other than RGB is used, the number, location and/or width of the color bands may be different. Other than that there is no principal difference to RGB color space.

The first imaged spectrum 202 does not include the fluorescence-excitation light and the fluorescence emission spectrum of the at least one fluorophore 116, 118. Thus, the first imaged spectrum 202 may include at least one stop band 210 which coincides with the fluorescence emission of the at least one fluorophore of which fluorescence is to be recorded by the fluorescence-light color camera 111. The stop band 210 is e.g. created by the white-light filter 188. The number, width, and/or location of stop bands 210 depend on the number and types of fluorophores to be observed in the object 106.

At reference numeral 220, a second imaged spectrum 222 is shown as it may be recorded by the fluorescence-light color camera 111 and/or represented in the digital fluorescence-light color image 112, respectively. Just by way of example, the color space in which the second imaged spectrum 222 is recorded is also an RGB color space. The sensitivities of the sensors of the fluorescence-light color camera 111 in the different primary colors 204, 206, 208 are tuned to result in a sensitivity across the visible spectrum 212 which is as constant as possible.

The spectra 202, 222 do not need to be recorded in the same color space, although this is preferred.

The second imaged spectrum 222 may comprise one or more passbands 224. The number, location and/or width of the passbands depends on the number and types of fluorophores used. The at least one passband 224 preferably corresponds to the at least one stop band 210. The at least one passband is e.g. generated by the fluorescence-light filter 190.

The first imaged spectrum 202 and the second imaged spectrum 222 are complementary to one another. They preferably complete each other to cover the entire or most of the visible spectrum 212.

Each passband 224 of the second imaged spectrum 222 preferably overlaps the fluorescence-emission spectrum 226, 228 of a fluorophore 116, 118, of which fluorescence is to be recorded, and overlap one or more primary colors 204, 206, 208 of the color spectrum. For example, the fluorescence-emission spectrum 226 of one fluorophore 116 may overlap all three primary colors 204, 206, 208. The sensor of the fluorescence-light camera 111 recording the color band 204 only records a small part 230 of the fluorescence-emission spectrum 226. The sensor of the fluorescence-light camera 111 recording the color band 206 records a majority 232 of the fluorescence-emission spectrum 226, whereas the sensor of the fluorescence-light camera 111 recording the color band 208 again only records a small part 236 of the fluorescence-emission spectrum 226.

The fluorescence spectrum 228, if present in a particular case, in contrast is only recorded by the sensor of the fluorescence-light camera 111 recording the color band 208.

The color conversion function 140 is applied to both the digital white-light color image 114 and the digital fluorescence-light color image 112 to obtain the digital output color image 160 of which the spectrum is indicated at reference numeral 250 in Fig. 2. In essence, the spectrum 252 of the digital output image 160 results in a combination of the first imaged spectrum 202 and the second imaged spectrum 222.

Greater accuracy may be achieved in that the individual spectra 202 and 222 are treated as a single, combined multispectral spectrum which has a number of color bands that corresponds to the sum of color bands in the first imaged spectrum 202 and the second imaged spectrum 222. In other words, the digital fluorescence-light color image 112 and the digital white-light color image 114 are jointly processed as a single (virtual) multispectral image.

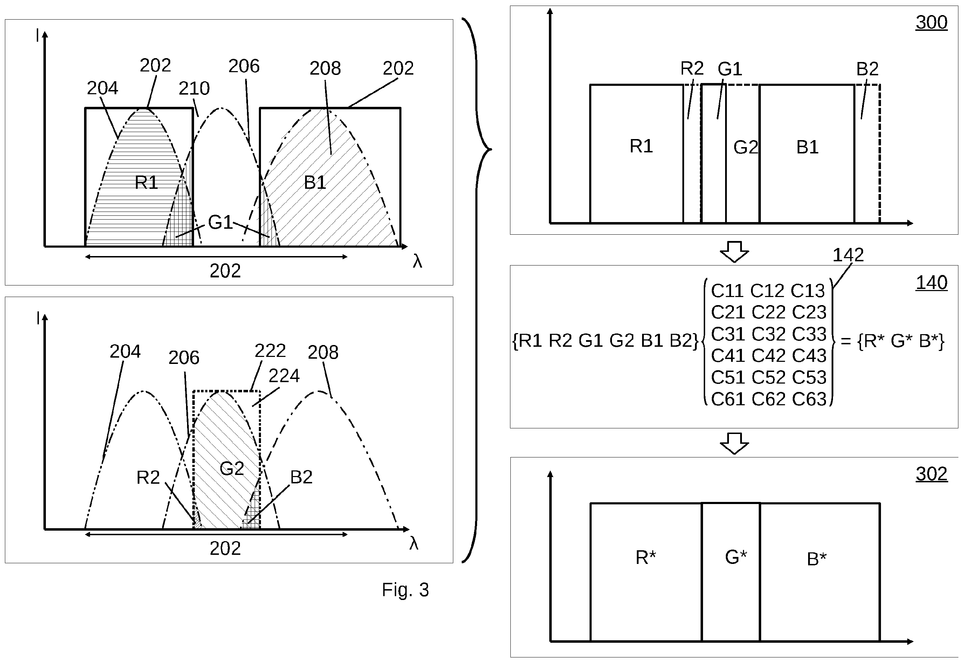

This is explained in the following with reference to Fig. 3. For simplicity's sake, Fig. 3 shows the spectra of Fig. 2 in an RGB color space. Only a single passband 224 or stopband 210 is shown.

The digital white-light color image 114 recorded in the first imaged spectrum 202 is composed of signals R1, G1 and B1, each representing the intensity I in the respective color band. Due to the stop band 210, the signal G1 results from two separated wavelengths bands. The sensor recording G1 however cannot distinguish between these two wavelength bands.

The digital fluorescence-light color image 112 recorded in the second imaged spectrum 222 is composed of signals R2, G2, B2 in each color band.

As the first and second imaged spectrum 202, 222 are complementary to each other, each color band is split into two signals, respectively, each signal coming from a different camera 110, 111 and representing or being equivalent to a sub-band of the color band. The color band 204 is split into R1 and R2, respectively. The color band 206 is split into G1 and G2, respectively, and the color band 208 is split into B1 and B2 respectively. Preferably, there is no overlap between the various signals in a color band.

Thus, even if the first and second imaged spectrum 202, 222 are recorded in the same color space using the same type of color cameras, a multispectral image of the object 106 results, which is composed in this case of six color bands R1, R2, G1, G2, B1, B2. This is shown schematically at reference numeral 300. It is to be noted that this is independent of whether the fluorescence camera actually records fluorescence emission or reflectance.

In other words, at least one color band 204, 206, 208 of the color space at least of the digital output color image 160 is subdivided into two sub-bands R1, R2, G1, G2, B1, B2, wherein one of the two sub-bands is comprised in the digital fluorescence-light color image 112 and the other of the two sub-bands is comprised in the digital white-light color image 114. Preferably, the two sub-bands in a color band do not overlap. They are preferably complementary. Most preferably, they, together, at least almost complete the respective color band. A sub-band may comprise or consist of two separated spectral bands. Each sub-band may be considered as being itself a color band.

The color conversion function 140 represents the subdivision of the color bands into the sub-bands R1, R2, G1, G2, B1, B2 that is determined by the stop-band(s) 210 and the pass-band(s) 224. As the widths and/or locations of the sub-bands determine how much light will be gathered by the respective camera 110, 111 in that sub-band, the color conversion function 140 needs to be adjusted for each different filter setting of the color separation assembly 176.

Thus, the digital white-light image 112 and the digital fluorescence-image 114 may be processed together as an image composed of the union of color bands in the two images 112 and 114. This provides improved color resolution.

The color conversion function 140 may be applied in the form of a linear transformation using the color conversion matrix 142 to generate the digital output color image 160.

If the digital output color image 160 is represented in an RGB color space, a 6x3 or 3x6 color conversion matrix may be applied to the combined digital fluorescence-light and white light image 112, 114 or their constituent signals or color space coordinates R2, G2, B2, R1, G1, B1, respectively to arrive at RGB signals R*, G*, B*. The matrix coefficients C11, C12, ..., C63 may be determined by experiment.

As a result of applying the color conversion function 302, an output spectrum 302 is obtained, which is represented by three RGB signals or color space coordinates.

The above transformation is applied at each image location or pixel.

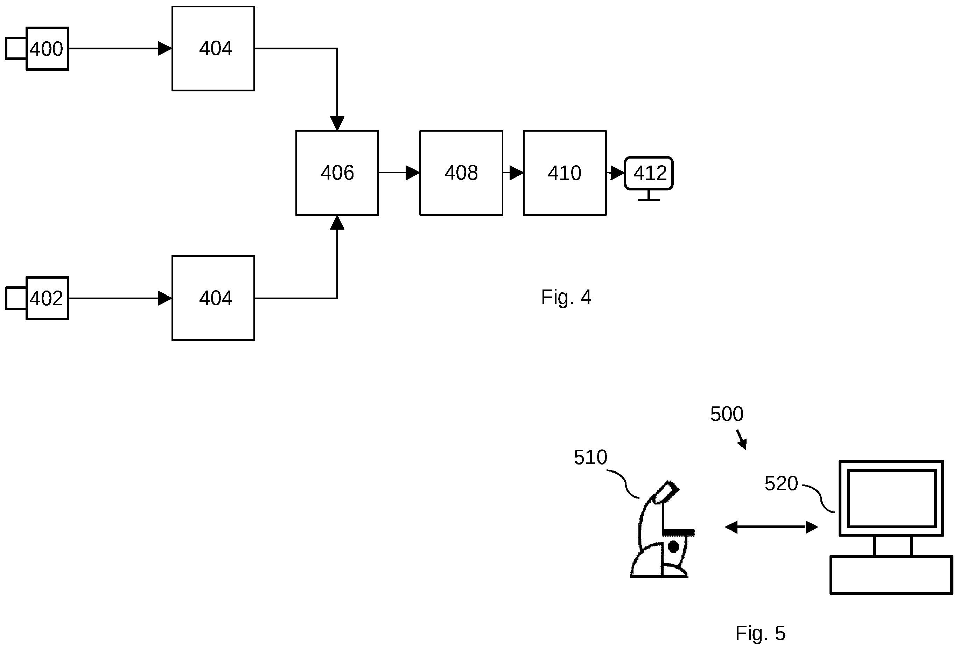

Fig. 4 shows a schematic representation of the imaging method that may be implemented as a computer-implemented method running e.g. on the image processor 170.

At optional step 400, a digital white-light color image 114 is recorded, using e.g. the white-light color camera 110. At optional step 402, a digital fluorescence-light color image 112 is recorded, using e.g. the fluorescence-light camera 111. Steps 400 and 402 are optional because the images 112, 114 may be retrieved from memory. As explained above, the cameras 110, 111 are synchronized with respect to exposure time and locked to one another with respect to gain, i.e. maintain the same gain ratio. Gamma may be set to a fixed value and all automatic color adjustments are preferably turned off.

At optional step 404, the respective image 112, 114 is demosaiced.

At optional step 406, one or both images 112 or 114 are matched and registered so that identical image features are represented in each image 112, 114 geometrically identically with respect to size and orientation.

At step 408, color conversion is performed. The images 112, 114 may be linearily transformed, e.g. using a color conversion matrix 142 to obtain the digital output color image 160.

At step 410, further post-processing may be carried out. For example, the digital output color image 160 may be homogenized, its contrast may be enhanced and/or color-space conversions such as from RGB to sRGB, and/or gamma corrections may be performed. At step 412, the digital output image 160 is displayed.

Some embodiments relate to a microscope comprising a system as described in connection with one or more of the Figs. 1 to 4. Alternatively, a microscope may be part of or connected to a system as described in connection with one or more of the Figs. 1 to 4.

Fig. 5 shows a schematic illustration of a system 500 configured to perform a method described herein. The system 500 comprises a microscope 510 and a computer system 520. The microscope 510 is configured to take images and is connected to the computer system 520. The computer system 520 is configured to execute at least a part of a method described herein. The computer system 520 may be configured to execute a machine learning algorithm. The computer system 520 and microscope 510 may be separate entities but can also be integrated together in one common housing. The computer system 520 may be part of a central processing system of the microscope 510 and/or the computer system 520 may be part of a subcomponent of the microscope 510, such as a sensor, an actor, a camera or an illumination unit, etc. of the microscope 510.

The computer system 520 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 520 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transition-ary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### REFERENCE NUMERALS

- 100: medical observation device
- 101L: stereoscopic subassembly for the left channel
- 101R: stereoscopic subassembly for the right channel
- 102: digital imaging system
- 104: eyepiece
- 106: object
- 107: biological tissue
- 110: white-light color camera
- 111: fluorescence-light color camera
- 112: digital fluorescence-light color image
- 114: digital white-light color image
- 116: fluorophore
- 118: second fluorophore
- 132: internal display
- 134: direct optical path
- 136: beam splitter
- 140: color conversion function
- 142: color conversion matrix
- 142a-142d: different color conversion matrices
- 160: digital output color image
- 170: image processor
- 172: output interface
- 174: objective
- 176: color-separation assembly
- 178: illumination assembly
- 179: illumination filter
- 180: illumination beam splitter
- 182: display
- 184: field of view
- 186: computer
- 188: white-light filter
- 190: fluorescence-light filter
- 192: dichroic beam splitter
- 194: memory
- 196: data transmission line
- 200: spectrum
- 202: first imaged spectrum
- 204, 206, 208: primary color or color band
- 210: stopband
- 212: visible spectrum or light range
- 220: spectrum
- 222: second imaged spectrum
- 224: passband
- 226: fluorescence-emission spectrum of a fluorophore
- 228: fluorescence emission spectrum of another fluorophore
- 230: part of fluorescence emission spectrum in color band 204
- 232: part of fluorescence emission spectrum in color band 206
- 236: part of fluorescence emission spectrum in color band 208
- 250: spectrum
- 252: spectrum of digital output image
- 300: multispectral spectrum resulting from first and second imaged spectrum
- 400: recording a digital white-light color image
- 402: recording a digital fluorescence-light color image
- 404: demosaicing
- 406: registering
- 408: color conversion
- 410: post-processing
- 412: displaying
- 500: system
- 510: microscope
- 520: computer system
- λ: wavelength
- I: intensity
- C11, C12, ..., C63: coefficients of the color conversion matrix
- RGB: example of a color space
- R1, G1, B1, R2, G2, B2, R*, G*, B*: (intensity) signals/color space coordinates in the respective color bands, or sub-bands of a color space

## Claims

1. Computer-implemented image processing method for generating a digital output color image (160) of an object (106), the method comprising the steps of:
- retrieving a digital white-light color image (114) of the object (106) recorded in a first imaged spectrum (202);
- retrieving a digital fluorescence-light color image (112) of the object (106) recorded in a second imaged spectrum (222), the second imaged spectrum (222) overlapping a fluorescence emission spectrum (226) of at least one fluorophore (116), the second imaged spectrum (222) being different from the first imaged spectrum (202), the first and the second imaged spectrum (202, 222) both overlapping the visible spectrum (212); and
- generating the digital output color image (160) by combining the digital fluorescence-light color image (112) and the digital white-light color image (114).

2. Computer-implemented image processing method according to claim 1, wherein the first imaged spectrum (202) and the second imaged spectrum (222) are complementary to one another.

3. Computer-implemented image processing method according to claim 2, wherein
the digital white-light color image (114) is represented in a first color space (RGB) using at least three first color bands (R, G, B);
the digital fluorescence-light color image (112) is represented in a second color space comprising at least three second color bands;
the digital output color image (160) is generated in a third color space comprising at least three third color bands;
the first color bands and the second color bands are mapped onto the third color bands of the digital output color image using a linear transformation and
wherein each color band of the first color bands and each color band of the second color bands is input as a separate color band into the linear transformation.

4. Computer-implemented image processing method according to claim 3, wherein
the linear transformation comprises a color conversion matrix which has a dimension of a number X times a number Y,
the number X being the sum of the quantity of the first color bands and the quantity of the second color bands,
the number Y being the quantity of color bands in the third color space.

5. Computer-implemented image processing method according to claim 3 or 4, wherein the first color space and the second color space are identical.

6. Computer-implemented image processing method according to claim 5, wherein the first imaged spectrum (202) comprises a first sub-band (R1, G1, B1) in a color band (204, 206, 208) of a color space (RGB), the second imaged spectrum (222) comprises a second sub-band (R2, G2, B2) in this color band and the first and the second sub-band are complementary to one another.

7. Computer-implemented image processing method according to claim 6, wherein a color band of the third color space comprises the first and the second sub-band.

8. Computer-implemented image processing method according to any one of claims 1 to 7, wherein the second imaged spectrum (222) comprises NIR wavelengths.

9. Computer-implemented image processing method according to any one of claims 1 to 8, wherein the digital fluorescence-light color image (112) and the digital white-light color image (114) are processed jointly as a digital multispectral image comprising at least five color bands.

10. Method (100) for operating a medical observation device (100), in particular a microscope or endoscope, the method comprising the steps of:
carrying out the computer-implemented image processing method according to any one of claims 1 to 9;
recording the digital white-light color image (114) using a white-light color camera (110); and
recording the digital fluorescence-light color image (112) using a fluorescence-light color camera (111).

11. Method according to claim 10, wherein the white-light color camera (110) and the fluorescence-light color camera (111) are synchronized with respect to at least one of exposure time and gain.

12. Image processor (170) for a microscope or endoscope, the image processor (170) being configured to:
retrieve a digital white-light color image (114) of an object (106) recorded in a first imaged spectrum (202);
retrieve a digital fluorescence-light color image (112) of the object (106) recorded in a second imaged spectrum (222), the second imaged spectrum (222) overlapping a fluorescence emission spectrum (226) of at least one fluorophore (116), the second imaged spectrum (222) being different from the first imaged spectrum (202), the first and the second imaged spectrum (202, 222) both overlapping the visible spectrum (222); and
generate a digital output color image (160) by combining the digital fluorescence-light color image (112) and the digital white-light color image (114).

13. Medical observation device (100), such as a microscope or endoscope, comprising
an image processor (170) according to claim 12;
a fluorescence-light color camera (111) configured to record the digital fluorescence-light color image (112); and
a white-light color camera (110) configured to record the digital white-light color image (114).

14. Use of a fluorescence-light color camera (111) in a medical observation device (100) to record part (222) of the spectrum (250) of a digital output color image (160) generated by the medical observation device (100), the medical observation device (100) further comprising a white-light color camera (110) configured to record another part (202) of the spectrum (250) of the digital output color image (160).

15. A computer program product or a computer-readable medium comprising instructions which, when the program is executed by a computer, cause the computer to carry out the method of any one of claims 1 to 9.
